# EUROPEAN PATENT APPLICATION

(11) **EP 3 884 771 A1**
(43) Date of publication of application: **29.09.2021**
(21) Application number: 19886358.1
(22) Date of filing: 19.11.2019
(51) Int. Cl.: A01K 67/027, C12N 5/071

(54) **COMPOSITION FOR RECONSTITUTING HUMAN SKIN TISSUE HAVING HAIR FOLLICLES, HUMAN SKIN TISSUE MODEL ANIMAL, AND PRODUCTION METHOD THEREOF**

(30) Priority: 19.11.2018 JP 2018216875
(71) Applicant: Shiseido Company, Ltd., Chuo-ku Tokyo 104-0061 (JP)
(72) Inventor: YOSHIDA, Yuzo, Tokyo 104-0061 (JP); SOMA, Tsutomu, Tokyo 104-0061 (JP); KISHIMOTO, Jiro, Tokyo 104-0061 (JP); MATSUZAKI, Takashi, Matsue-shi, Shimane 690-8504 (JP)
(74) Representative: Cabinet Beau de Loménie
(86) International application number: PCT/JP2019/045303
(87) International publication number: WO 2020/105643

(57) **Abstract**

The present invention provides: a composition for reconstituting human skin tissue having hair follicles, the composition characterized by containing human epidermal cells and human dermal cells, the human dermal cells containing cell groups of human hair papilla derived from non-embryos via spheroid formation; and a human skin tissue model animal to which said composition is applied. The invention also provides a method for producing said composition and animal.

## Description

### FIELD

The present invention relates to a composition for reconstitution of hair follicle-containing human skin tissue and to a method for producing it. The invention further relates to an animal model of hair follicle-containing human skin tissue to which a composition for reconstitution of hair follicle-containing human skin tissue has been applied, and to a method for producing it.

### BACKGROUND

Hair is produced by hair follicles present in the skin. A hair follicle is a panniculus surrounding a hair, and is composed of a hair matrix (hair matrix cells), an inner root sheath and an outer root sheath that derive from the ectoderm, and a dermal sheath and dermal papilla that derive from the mesoderm. The hair matrix cells surrounding the dermal papilla undergo repeated division when induced by nutrients and proteins supplied from the dermal papilla, forming hair by a process of keratinization.

Hair thinning and alopecia occur due to problems in the development of hair. Hair thinning and alopecia include conditions such as premature alopecia, alopecia areata and telogen effluvium, with premature alopecia being most common. Premature alopecia is caused in males primarily by the influence of male sex hormones, and is also known as androgenetic alopecia. Hair is regenerated by repetition of the hair cycle which consists of an anagen phase, catagen phase and telogen phase. Androgenetic alopecia is a symptom caused by a shortened anagen phase during the hair cycle, increasing the proportion of thin, short hairs.

The external preparation minoxidil and the oral drug finasteride are the main treatments for premature alopecia used at the current time. These treatments are both confirmed to be effective and safe, but are not always effective for all cases of premature alopecia.

Another treatment for premature alopecia is autologous hair transplantation. In autologous hair transplantation, hair containing hair roots, harvested from the temporal regions or occipital region, is transplanted into hair loss sites of the same individual. This technique, however, merely replants hair of the individual into different sites and does not increase the total number of hairs.

A number of regenerative medicine techniques for various diseases have been developed in recent years, and new techniques are also being developed in the field of hair regeneration. For development of new hair regeneration techniques it is important to establish systems for properly evaluating hair growth effects, and several research groups have developed reconstituted skin with hair follicles (see PTL 1 and NPLs 1 and 2, for example).

### [CITATION LIST]

### [PATENT LITERATURE]

[PTL 1] Japanese Unexamined Patent Publication No. 2015-97524

### [NON PATENT LITERATURE]

[NPL 1] Ehama R., et al., Hair follicle regeneration using grafted rodent and human cells. J Invest Dermatol. 2007 Sep; 127(9):2106-2115. Epub 2007 Apr 12.
[NPL 2] Lee LF., et al., A simplified procedure to reconstitute hair-producing skin. Tissue Eng. Part C Methods. 2011 Apr; 17(4):391-400

### SUMMARY

### [TECHNICAL PROBLEM]

While treatment methods using hair follicle-derived cells continue to be developed in the field of hair regeneration, for proper evaluation of the effects of the developed treatment methods it is important to establish evaluation systems with reconstituted human skin containing human hair follicles, that can maintain their structures over long periods. At the current time, however, it is still difficult to provide reconstituted human skin containing human hair follicles with satisfactory reproducibility, using materials that can be obtained in a stable manner.

It is therefore an object of the present invention to provide a composition for reconstitution of human hair follicle-containing human skin tissue that has satisfactory reproducibility when using materials that can be obtained in a stable manner, as well as a method for producing it. It is another object of the invention to provide an animal model of hair follicle-containing human skin tissue to which a composition for reconstitution of hair follicle-containing human skin tissue has been applied, and to a method for producing it.

### [SOLUTION TO PROBLEM]

As a result of ardent research, the present inventors have completed this invention upon finding that it is possible to reconstitute hair follicle-containing human skin tissue in a stable manner with satisfactory reproducibility by including in the composition a non-fetal-derived human dermal papilla cell aggregation that has undergone spheroid formation. Specifically, the present invention encompasses the following inventions.
[1] A composition for reconstitution of hair follicle-containing human skin tissue, comprising:
   human epidermal cells; and
   human dermal cells,
   wherein the human dermal cells include a cell population of non-fetal-derived human dermal papilla cells that have undergone spheroid formation.
[2] The composition according to [1], wherein the non-fetal-derived human dermal papilla cells are non-fetal-derived human dermal papilla cells stimulated with a Wnt signal activating agent.
[3] The composition according to [2], wherein the Wnt signal activating agent is a Wnt ligand family protein or a Wnt pathway agonist.
[4] The composition according to [3], wherein the Wnt ligand family protein is selected from the group consisting of Wnt1, Wnt2, Wnt2b, Wnt3, Wnt3a, Wnt4, Wnt5a, Wnt5b, Wnt6, Wnt7a, Wnt7b, Wnt8a, Wnt8b, Wnt9a, Wnt9b, WntlOa, WntlOb, Wnt11, Wnt16 and their combinations.
[5] The composition according to [3], wherein the Wnt pathway agonist is CHIR99021 or BIO.
[6] The composition according to any one of [1] to [5], wherein the human dermal cells include human fibroblasts.
[7] A hair follicle-containing human skin tissue animal model, wherein a composition according to any one of [1] to [6] has been applied to a non-human mammal.
[8] A method for producing a composition for reconstitution of hair follicle-containing human skin tissue, wherein the method comprises:
   (1) a step of preparing spheroids containing non-fetal-derived human dermal papilla cells, and
   (2) a step of mixing and culturing the spheroids, with human epidermal cells and human dermal cells.
[9] The method according to [8], wherein step (1) is carried out in the presence of a Wnt signal activating agent.
[10] The method according to [9], wherein the Wnt signal activating agent is a Wnt ligand family protein or a Wnt pathway agonist.
[11] The method according to [10], wherein the Wnt ligand family protein is selected from the group consisting of Wnt1, Wnt2, Wnt2b, Wnt3, Wnt3a, Wnt4, Wnt5a, Wnt5b, Wnt6, Wnt7a, Wnt7b, Wnt8a, Wnt8b, Wnt9a, Wnt9b, WntlOa, WntlOb, Wnt11, Wnt16 and their combinations.
[12] The method according to [10], wherein the Wnt pathway agonist is CHIR99021 or BIO.
[13] The method according to any one of [8] to [12], wherein step (1) is carried out by the hanging drop method.
[14] The method according to any one of [8] to [13], wherein the non-fetal-derived human dermal papilla cells are adult-derived human dermal papilla cells.
[15] The method according to any one of [8] to [14], wherein the human dermal cells include human fibroblasts.
[16] The method according to any one of [8] to [15], wherein the human epidermal cells are human epidermal cells stimulated by an extracellular matrix protein or its derivative.
[17] The method according to [16], wherein the extracellular matrix protein includes laminin or its fragment.
[18] The method according to any one of [8] to [17], wherein step (2) is a step of culturing on a culture substrate comprising a porous film.
[19] A method for producing a hair follicle-containing human skin tissue animal model, comprising:
   a step of applying a composition obtained by the method according to any one of [8] to [18] to a non-human mammalian animal.
[20] A composition for reconstitution of hair follicle-containing human skin tissue, obtained by the method according to any one of [8] to [18].
[21] A hair follicle-containing human skin tissue animal model obtained by the method according to [19].

### [ADVANTAGEOUS EFFECTS OF INVENTION]

According to the invention it is possible to provide a composition that allows hair follicle-containing human skin tissue to be reconstituted in a stable and reproducible manner, as well as a method for producing it. It is also possible to provide a hair follicle-containing human skin tissue animal model to which the composition has been applied, and a method for producing it. The evaluation system provided by the invention allows the effects of drugs under development, regenerative medicine technology or beauty technology, and particularly hair growth effects, to be evaluated with high reproducibility.

### BRIEF DESCRIPTION OF DRAWINGS

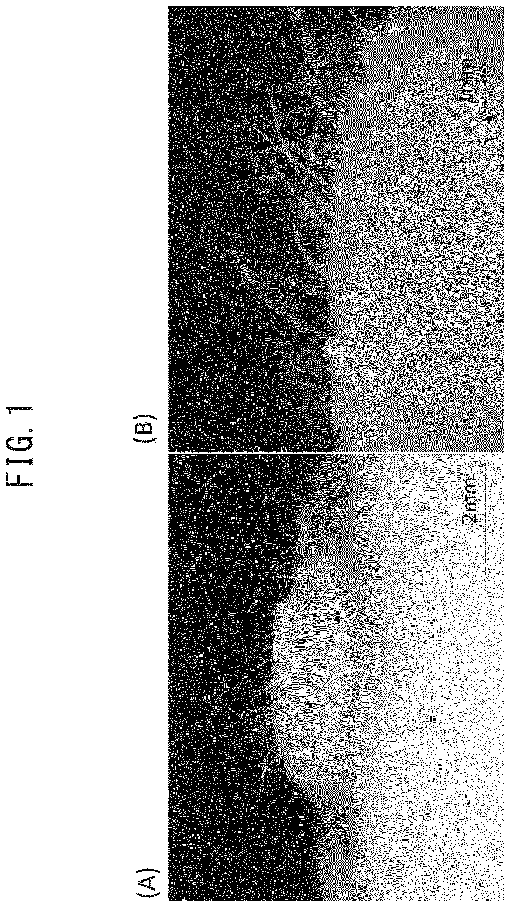

Fig. 1 is a photograph showing SCID mouse skin obtained by applying a composition of the invention according to an embodiment. (A) External photograph of reconstituted skin. (B) Magnified view of hair-growing portion of (A).

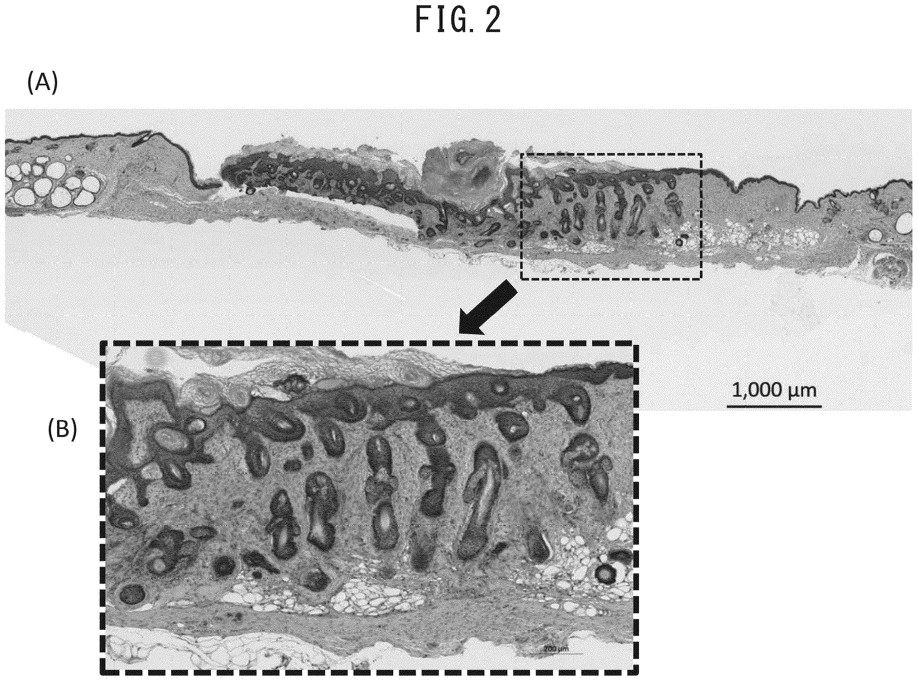

Fig. 2 shows tissue analysis results for SCID mouse skin obtained by applying a composition of the invention according to an embodiment. (A) Tissue section of H&E-stained reconstituted skin. (B) Magnified view of dotted line portion of (A). Numerous hair follicles are seen within the reconstituted skin.

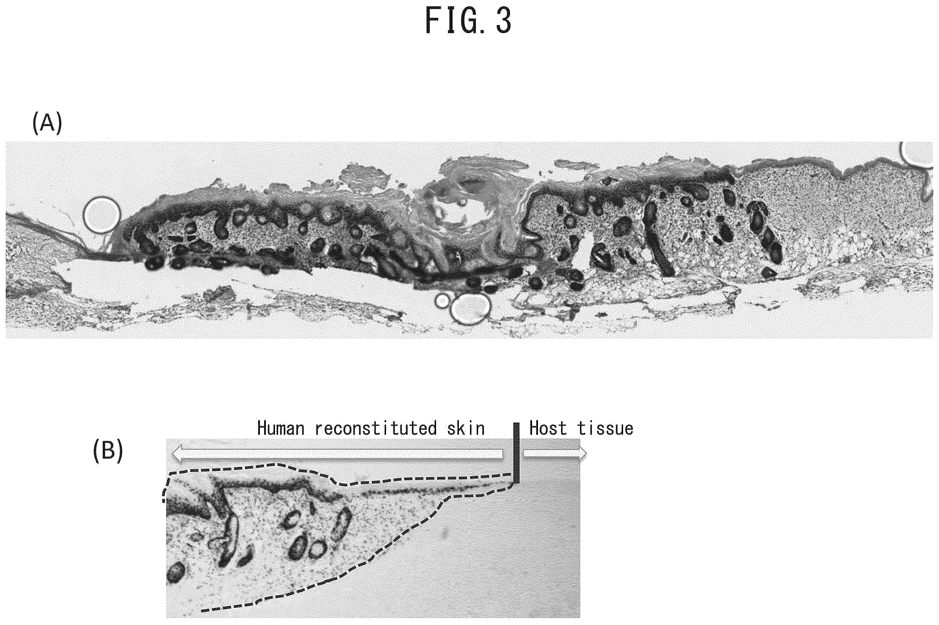

Fig. 3 shows tissue analysis results for SCID mouse skin obtained by applying a composition of the invention according to an embodiment. (A) Stained image of tissue section of reconstituted skin stained by *in situ* hybridization using a human Alu sequence-specific probe. (B) Image of the boundary portion between reconstituted skin and host tissue, stained by *in situ* hybridization. The reconstituted skin is shown to consist of human cells, indicating that it is human reconstituted skin.

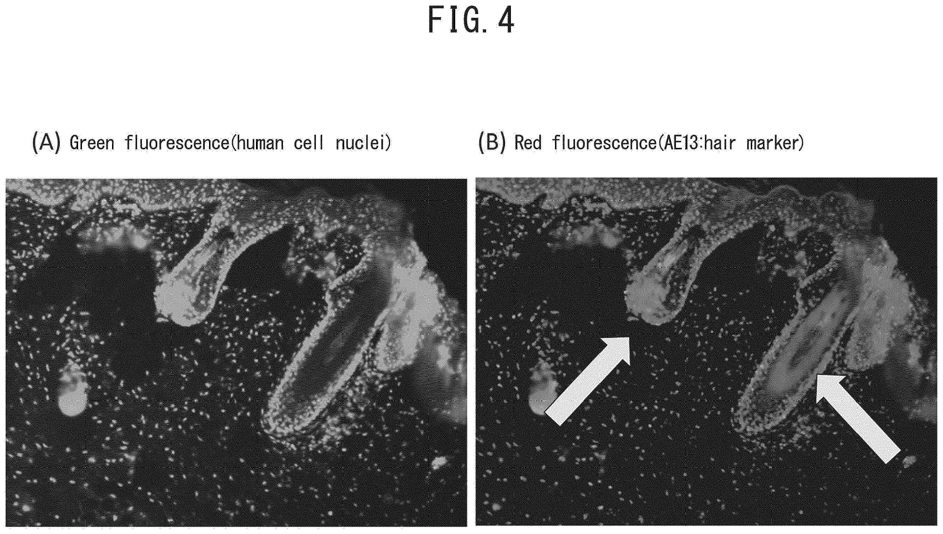

Fig. 4 shows tissue analysis results for SCID mouse skin obtained by applying a composition of the invention according to an embodiment. (A) Stained image of tissue section of reconstituted skin stained by *in situ* hybridization using a human Alu sequence-specific probe. (B) Stained image of reconstituted skin tissue section, from hair shaft stained with antibody that specifically recognizes AE13. The hair and hair follicles are shown to consist of human cells, indicating that they are human hair follicles.

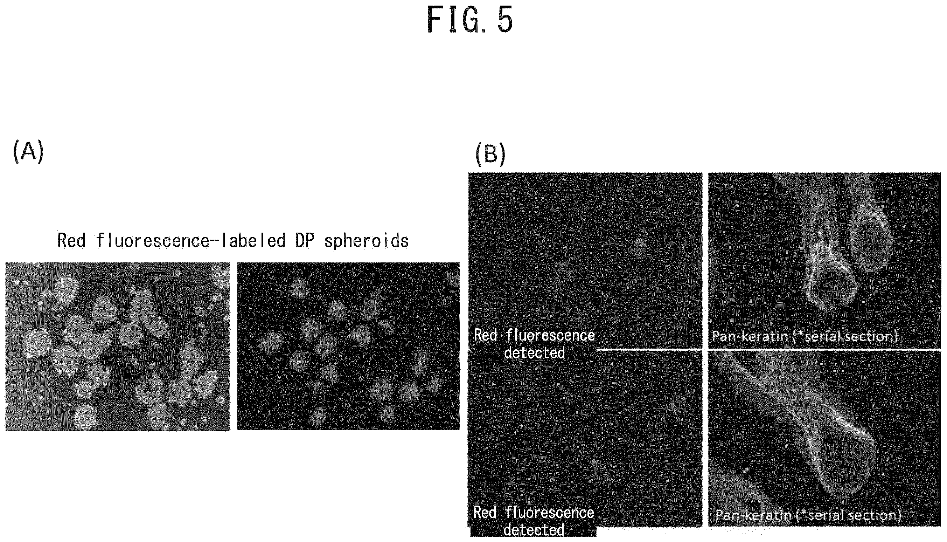

Fig. 5 shows dermal papilla (DP) spheroids stimulated with Wnt pathway agonist (CHIR99021). (A) Dermal papilla (DP) spheroids labeled with red fluorescent dye PKH-26 (left: differential interference contrast image, right: fluorescent image). (B) Fluorescent image of reconstituted skin tissue section. Left column: red fluorescent image (DP spheroids), right column: fluorescent image with anti-Pan-keratin antibody (green) and DAPI (blue). The upper and lower levels each show images of the same tissue section. It is seen that the hair follicles formed in the reconstituted skin are derived from dermal papilla (DP) spheroids stimulated with Wnt pathway agonist (CHIR99021). It is thus demonstrated that dermal papilla (DP) spheroids stimulated with Wnt pathway agonist (CHIR99021) are essential for formation of human hair follicles in reconstituted skin.

### DESCRIPTION OF EMBODIMENTS

Embodiments for carrying out the invention will be described in detail below, with the understanding that the technical scope of the invention is not limited only to these embodiments. Unless otherwise specified, the descriptions of the embodiments for carrying out the invention also apply to the composition for reconstitution of hair follicle-containing human skin tissue and the method for producing it, and to the hair follicle-containing human skin tissue animal model and the method for producing it.

### 1. Composition for reconstitution of hair follicle-containing human skin tissue

According to one embodiment, the composition for reconstitution of hair follicle-containing human skin tissue of the invention comprises:
human epidermal cells; and
human dermal cells,
wherein the human dermal cells include a cell population of non-fetal-derived human dermal papilla cells that have undergone spheroid formation.

The phrase "composition which reconstitutes hair follicle-containing human skin tissue", as used herein, means a composition having the ability to reconstitute human skin-like tissue with hair follicle structures when applied onto skin, at the site where it is applied.

The term "hair follicle" (HF) generally refers to the panniculus surrounding a hair, including the hair matrix cells, as epithelial cells, the inner root sheath, the outer root sheath, and the dermal sheath and dermal papilla, which are mesenchymal cells. Application of the composition of the invention can reconstitute such human skin-like tissue with human hair follicles at the site where it has been applied. Hair growth also occurs from the reconstituted hair follicles.

For the purpose of the invention, "epidermal cells" are cells forming the epidermis, and they include cells at different stages of differentiation (primarily keratinocytes). In living bodies, the epidermis is formed of epidermal cells at different stages of differentiation that are overlaid in a laminar fashion. The deepest part of the epidermis is called the basal lamina, where cylindrical cells form a single layer. With progressive stages of differentiation, epidermal cells migrate to the outer layer while changing to a flat form. The basal lamina is located at the interface with the dermis, and the stratum spinosum is present on its upper layer. Epidermal cells that have progressed in differentiation from the stratum spinosum form a granular layer with keratohyalin granules and lamellar granules. The granular layer is composed of about 2 or 3 layers in biological tissue. When the differentiation stage progresses even beyond the granular layer, the cell nuclei are lost and the stratum corneum is formed. The epidermis also includes melanocytes, Langerhans cells and Merkel cells in addition to epidermal cells. The composition of the invention may also include other cells included in the epidermis, in addition to epidermal cells.

According to one embodiment, the epidermal cells in the composition of the invention are human-derived epidermal cells. The human epidermal cells may be commercially available human epidermal cells, and they may be primary human epidermal cells obtained by fine cutting of biological tissue and treatment with proteases such as collagenase or trypsin, or fibroblasts grown by subculturing of the obtained primary human epidermal cells. According to one embodiment, epidermal cells included in the composition of the invention may be derived from a human fetus, neonate (0 to 2 years of age, for example), juvenile (3 to 17 years of age, for example) or adult (18 or older, 20 or older, 25 or older, 30 or older, 35 or older or 40 or older, for example). According to another embodiment, epidermal cells included in the composition of the invention may be epidermal cells differentiated from pluripotent stem cells or tissue stem cells. Pluripotent stem cells may be iPS cells, ES cells or Muse cells, or epidermal cells obtained by inducing differentiation from these pluripotent stem cells by a publicly known method.

According to one embodiment, epidermal cells included in the composition of the invention are epidermal cells stimulated by an extracellular matrix protein or its derivative. Examples of extracellular matrix proteins include laminin, nidogen, tenascin, thrombospondin, fibronectin, vitronectin, collagen and elastin. An extracellular matrix protein for stimulation of epidermal cells may be a natural extracellular matrix protein or its derivative (recombinant form). It may also be an extracellular matrix protein fragment having a function of stimulating epidermal cells. Epidermal cells stimulated by an extracellular matrix protein are preferably included in the composition of the invention, since this will increase the ability of the composition to reconstitute human skin-like tissue with human hair follicles.

According to one embodiment, the extracellular matrix protein for stimulation of epidermal cells includes laminin or a fragment thereof. Laminin is a protein composed of a combination of α chains (LAMA1, LAMA2, LAMA3, LAMA4, LAMA5), β chains (LAMB 1, LAMB2, LAMB3, LAMB4) and γ chains (LAMC1, LAMC2, LAMC3), and 17 combinations (laminins 1 to 15, laminins 212/222 and laminin 522) are known as of the current time. For example, the combination of α chains, β chains and γ chains in the laminins LAMA5, LAMB1 and LAMC1 is known as "laminin 511". Laminin 511 is also known by the alternate name "laminin 10". According to one embodiment, the extracellular matrix protein for stimulation of epidermal cells may be a laminin fragment, such as laminin 511-E8 fragment.

As used herein, "dermal cells" are cells included in the dermis that is present between the epidermis and subcutaneous tissue of skin, and they consist mostly of fibroblasts. The dermis is composed of fibroblasts, with collagen, elastic fibers (elastin), extracellular matrix and hyaluronic acid as well.

According to one embodiment, the dermal cells in the composition of the invention are human-derived dermal cells. The human dermal cells may be commercially available human dermal cells, or they may be primary human dermal cells obtained by fine cutting of biological tissue and treatment with proteases such as collagenase or trypsin, or dermal cells grown by subculturing of the obtained primary human dermal cells. According to one embodiment, dermal cells included in the composition of the invention may be derived from a human fetus, neonate (0 to 2 years of age, for example), juvenile (3 to 17 years of age, for example) or adult (18 or older, 20 or older, 25 or older, 30 or older, 35 or older or 40 or older, for example). According to another embodiment, dermal cells included in the composition of the invention may be dermal cells differentiated from pluripotent stem cells or tissue stem cells. Pluripotent stem cells may be iPS cells, ES cells or Muse cells, or dermal cells obtained by inducing differentiation from these pluripotent stem cells by a publicly known method.

The swelling portion of the deepest part of the skin interior of hair is called the hair bulb, while the portion consisting of mesenchymal cells at the center portion of the hair bulb is called the dermal papilla (DP). The cells composing the dermal papilla are referred to as "dermal papilla cells". Capillaries and nerves infiltrate the dermal papilla, bringing in food nutrients and oxygen and regulating generation and growth of hair. Hair matrix cells are in contact with the dermal papilla, with hair being produced in this region. Hair matrix cells take up nutrients and oxygen from the capillaries in the dermal papilla, undergoing repeated division and forming hair.

According to one embodiment, the dermal papilla cells in the composition of the invention are human-derived dermal papilla cells. According to one embodiment, the human dermal papilla cells can be grown by isolating human hair follicles (preferably human scalp hair follicles), and further isolating and culturing tissue in the dermal papilla region of the isolated human hair follicles. As a specific example, scalp skin is cut to strips of about 5 mm and rinsed with phosphate buffered saline (PBS), with protease or surgical treatment as necessary, the epidermis layer and dermis layer are removed leaving only the subcutaneous fat layer, and then the hair follicles are isolated using physical means such as forceps. The hair bulbs are removed from the hair follicles, exposing the dermal papillae from below the hair bulbs and allowing isolation of the dermal papilla cells.

Culturing of the dermal papilla cells may be primary culturing and subculturing with commercially available nutrient medium used for culturing of animal cells, used either directly or after modification. Substitute medium to be used for culturing of the dermal papilla cells may be fetal bovine serum-containing Dulbecco's modified Eagle medium, Chang's medium, or MesenPRO medium (Thermo Fisher). If necessary, the medium may contain added cell growth factors, hormones or other trace nutrients. Specific components of this kind include transferrin, insulin, triiodothyronine, glucagon, hydrocortisone, testosterone, estradiol, progesterone and selenium.

The human dermal papilla cells to be included in the composition of the invention are non-fetal-derived human dermal papilla cells, and they may be dermal papilla cells derived from a juvenile (3 to 17 years of age, for example), or an adult (18 years or older, for example). According to the invention it is possible to reconstitute hair follicle-containing human skin tissue even using non-fetal-derived human dermal papilla cells, which have been difficult to use in the prior art.

According to another embodiment, dermal papilla cells included in the composition of the invention may be dermal papilla cells differentiated from pluripotent stem cells or tissue stem cells. Pluripotent stem cells may be iPS cells, ES cells or Muse cells, or dermal papilla cells obtained by inducing differentiation from these pluripotent stem cells.

According to one embodiment, the composition of the invention includes a cell population consisting of non-fetal-derived human dermal papilla cells that have undergone spheroid formation. As used herein, "spheroid" refers to a spherical cell aggregate comprising cells that have aggregated or agglomerated together. Examples of methods for forming the spheroids include a method of forming spheroids while floating the cells in a U-bottom well having a surface with low cell adhesion, a method of forming spheroids while adhering the cells to a surface having a particular concavoconvex structure, the hanging drop method, and a method of creating spheroids by culturing large amounts of the cells while rotating a cell culture chamber that contains a medium. The cell population of the human dermal papilla cells in the composition of the invention preferably consists of spheroids formed by the hanging drop method. The efficiency for reconstitution of the hair follicle-containing human skin tissue can be increased if a cell population of non-fetal-derived human dermal papilla cells that have undergone spheroid formation are included in the composition.

The number of human dermal papilla cells in each cell population of human dermal papilla cells that have formed spheroids may be 1 × 10 to 1 × 10⁵, preferably 1 × 10² to 1 × 10⁴ and more preferably 1 × 10² to 5 × 10³, for example. The mean diameter of the cell population of human dermal papilla cells that have formed spheroids is 20 µm to 1000 µm, preferably 50 µm to 300 µm and more preferably 70 µm to 200 µm.

According to one embodiment, the non-fetal-derived human dermal papilla cells in the composition of the invention are non-fetal-derived human dermal papilla cells that have been stimulated with a Wnt signal activating agent. The Wnt signal is a series of actions that promote nuclear localization of β-catenin and exhibit function as a transcription factor. The signal includes a cascade due to intercellular interaction, when for example, the proteinWnt3A secreted from certain cells acts on other cells, causing nuclear localization of intracellular β-catenin, and leading to activity as a transcription factor. The cascade brings about the initial phenomenon of organ assembly, of which epithelium mesenchymal interaction is a typical example. The Wnt signal is known to control various cell functions such as cell motility during cellular growth and differentiation, organogenesis and early development, by activation of three pathways, the β-catenin pathway, PCP pathway and Ca²⁺ pathway. It is utilized during culturing of ES cells, for the purpose of controlling differentiation, by the undifferentiated state-maintaining function of the Wnt signal (see Noburo Sato et al., Nature Medicine Vol.10, No.1, Jan. 2004). The efficiency of reconstitution of hair follicle-containing human skin tissue can be increased if non-fetal-derived human dermal papilla cells that have been stimulated by a Wnt signal activating agent are included in the composition of the invention.

The Wnt signal activating agent may be a Wnt ligand family protein or a Wnt pathway agonist. Examples of Wnt ligand family proteins include Wnt1, Wnt2, Wnt2b, Wnt3, Wnt3a, Wnt4, Wnt5a, Wnt5b, Wnt6, Wnt7a, Wnt7b, Wnt8a, Wnt8b, Wnt9a, Wnt9b, WntlOa, WntlOb, Wnt11 and Wnt16, and combinations of these Wnt ligand family proteins may also be used.

Examples of Wnt pathway agonists include glycogen synthase kinase-3 (GSK-3) inhibitor, R-spongins 1 to 4, and Norrin. Examples of GSK-3 inhibitors include CHIR99021, bis-indolo(indirubin) compounds (BIO) ((2'Z, 3'E)-6-bromoindirubin-3'-oxime), the acetoxime analog BIO-acetoxime(2'Z, 3'E)-6-bromoindirubin-3'-acetoxime), thiadiazolysine (TDZD) analogs (4-benzyl-2-methyl-1,2,4-thiadiazolysine-3,5-dione), oxothiadiazolysine-3-thione analogs (2,4-dibenzyl-5-oxothiadiazolysine-3-thione), thienyl α-chloromethyl ketone compounds (2-chloro-1-(4,4-dibromo-thiophen-2-yl)-ethanone), phenyl α-bromomethyl ketone compounds (α-4-dibromoacetophenone), thiazole-containing urea compounds (N-(4-methoxybenzyl)-N'-(5-nitro-1,3-thiazole-2-yl)urea), and GSK-3β peptide inhibitors such as H-KEAPPAPPQSpP-NH₂.

The amount of Wnt signal activating agent to be added is not particularly restricted and may be set as appropriate by a person skilled in the art. When CHIR99021 is used as the Wnt signal activating agent for dermal papilla cells, for example, an amount of 0.1 µM to 10 µM will allow stimulation, though there is no limitation to this amount.

The composition of the invention may also include biocompatible substances in addition to the cells mentioned above. Examples of biocompatible substances include, but are not limited to, water, physiological saline, phosphate buffer, cell culture media and biocompatible hydro gels (such as chitosan gel, collagen gel, gelatin, peptide gel, laminin gel and fibrin gel).

The composition of the invention is preferably prepared on a culture substrate comprising a porous film. The culture substrate comprising a porous film may be a cell culture insert, for example. The method for producing the composition of the invention will be described below.

The composition of the invention can be applied to a human or non-human mammal to reconstruct hair follicle-containing human skin tissue at the site of application.

### 2. Hair follicle-containing human skin tissue animal model

According to one embodiment, the composition of the invention is applied to a non-human mammal. A hair follicle-containing human skin tissue animal model can thus be obtained. Examples of non-human mammals include rats, mice, guinea pigs, marmosets, rabbits, dogs, cats, sheep, pigs, goats, monkeys, chimpanzees, and these same non-human mammalian animals with impaired immune systems, among which non-human mammalian animals with impaired immune systems are preferred. A method for producing a hair follicle-containing human skin tissue animal model of the invention will now be described.

The hair growth or hair promoting effect of a candidate substance can be evaluated by applying a candidate substance that exhibits a potential hair growth or hair promoting effect to a hair follicle-containing human skin tissue animal model of the invention, and observing the state of hair growth from hair follicles, or the state of hair follicle tissue, or a marker associated with hair growth. Examples of candidate factors include low molecular compounds, peptides, nucleic acids, proteins, mammalian animal (such as mouse, rat, pig, cow, sheep, monkey or human) cells, tissue extracts or cell culture supernatants, plant-derived compounds or extracts (such as galenical extracts or galenical-derived compounds), and microbial compounds or extracts, or culture products. For example, the effects on hair follicles to be formed and/or growth of the hair follicles may be evaluated based on differences such as the donor or the culturing conditions for the human dermal papilla cells to be included in the composition of the invention.

### 3. Method for producing composition for reconstitution of hair follicle-containing human skin tissue, and composition for reconstitution of hair follicle-containing human skin tissue obtained by the method

According to one embodiment, the method for producing a composition for reconstitution of hair follicle-containing human skin tissue according to the invention comprises:
(1) a step of preparing spheroids containing non-fetal-derived human dermal papilla cells, and
(2) a step of mixing and culturing the spheroids, human epidermal cells and human dermal cells.

In the method for producing the composition of the invention, the step of preparing spheroids that include non-fetal-derived human dermal papilla cells may employ, for example, a method of forming spheroids while floating the cells in a U-bottom well having a surface with low cell adhesion, a method of forming spheroids while adhering the cells to a surface having a particular concavoconvex structure, the hanging drop method, or a method of creating spheroids by culturing large amounts of the cells while rotating a cell culture chamber that contains a medium, with the hanging drop method being preferred.

The hanging drop method is a method in which droplets of a cell-containing culture solution is spotted onto the ceiling side of the culture dish cover, or droplets are formed from a special incubator (such as an Elplasia MPc (Kuraray, Japan)), and the cells in the culture solution are cultured in droplet form by surface tension. Culturing in this manner can minimize effects on the cells by contact with the culture substrate surface.

Spheroids including non-fetal-derived human dermal papilla cells, as well as human epidermal cells and human dermal cells, may be prepared to different cell numbers (or spheroid numbers) depending on the purpose, and for example, they may be mixed and cultured with a spheroid : human epidermal cells : human dermal cell ratio of 10 to 10,000 : 0.1 × 10⁶ to 100 × 10⁶ : 0.1 × 10⁶ to 100 × 10⁶, preferably 20 to 3000 : 1 × 10⁶ to 10 × 10⁶ : 1 × 10⁶ to 10 × 10⁶ and more preferably 50 to 1000 : 3 × 10⁶ to 6 × 10⁶ : 3 × 10⁶ to 6 × 10⁶.

According to one embodiment, step (1) is carried out in the presence of a Wnt signal activating agent. The human dermal papilla cells activated by the Wnt signal activating agent have even more accelerated hair follicle reconstitution. The time for treatment of the human dermal papilla cells with the Wnt signal activating agent may be 6 hours to 120 hours, preferably 12 hours to 108 hours, more preferably 24 hours to 96 hours and even more preferably 36 hours to 84 hours.

According to one embodiment, the human epidermal cells to be used in the method of the invention are human epidermal cells stimulated by an extracellular matrix protein or its derivative. The method of stimulating the human epidermal cells may be, for example, a method of culturing in medium containing the added extracellular matrix protein or its derivative, or a method of culturing on a culture substrate coated with the extracellular matrix protein or its derivative. It is preferably a method of culturing on a culture substrate coated with the extracellular matrix protein or its derivative. The coating method is not particularly restricted and may be any publicly known method. Reconstitution of the hair follicles can be further accelerated by using human epidermal cells that have been stimulated with an extracellular matrix protein or its derivative.

According to one embodiment, step (2) of the method of the invention is a step of culturing on a culture substrate comprising a porous film. The culture substrate comprising a porous film may be a commercially available cell culture insert, for example. The mean pore size of the porous film may be selected as appropriate, such as 0.01 µm to 100 µm, preferably 0.05 µm to 50 µm, more preferably 0.1 µm to 25 µm and most preferably 1 µm to 10 µm.

According to one embodiment, in step (2) a suspension comprising the mixed spheroids, human epidermal cells and human dermal cells is applied onto a culture substrate comprising a porous film and cultured for a predetermined time (for example, a time during which the liquid components including the medium are discharged, such as 0.5 hours to 3 hours). A sheet-like composition is thus formed on the culture substrate.

According to one embodiment, a composition for reconstitution of hair follicle-containing human skin tissue can be obtained by carrying out the method described above.

### 4. Method for producing hair follicle-containing human skin tissue animal model

The present invention provides a method for producing a hair follicle-containing human skin tissue animal model. According to one embodiment, the hair follicle-containing human skin tissue animal model is produced by a method that includes a step of applying the composition to a non-human mammalian animal.

According to one embodiment, the step of applying the composition of the invention to a non-human mammalian animal may be carried out, for example, by incising the skin at any site (such as the dorsal region) of the non-human mammalian animal to any size, and transplanting the composition into the site of incision. The size of the incision site is not particularly restricted and may be set as appropriate depending on the size and form of the composition to be applied (a suspension form or sheet form, for example). The depth of the incision site is not restricted since it will depend on the thickness of the skin of the non-human mammalian animal to which application is to be made. The composition of the invention may be applied at the site where the skin has been removed by incision, and sutured together with the skin surrounding the incision site, to transplant the composition. After application of the composition, it may be secured by any type of dressing material to prevent the composition from physically falling off.

### EXAMPLES

The present invention will now be explained in greater detail by examples, with the understanding that the invention is not limited in any way by the examples.

### <Example 1: Preparation of reconstituted skin>

### (1) Preparation of cells

Dermal papilla cells were obtained by isolating the dermal papilla region from human hair follicles (age 20 to 65, male) using a scalpel and forceps, setting it in a culture dish, and culturing with MesenPRO medium (Thermo Fisher). The human fibroblasts and epidermal cells were purchased as commercially available cells from Iwai Chemicals Co., and were prepared.

### (2) Cell preparation

After adding 3 µM CHIR99021 (Axon Medchem Co.) to the isolated human dermal papilla cells and culturing, spheroids were formed by the hanging drop method using Elplasia MPc (Kuraray, Japan).

The human epidermal cells were cultured in Epilife medium (Thermo Fisher) on a culture flask coated with iMatrix-511 (Nippi, Inc.).

The human fibroblasts were cultured in medium obtained by mixing DMEM medium and F12 medium at 3:1 ratio, and adding 5% FBS and 40 ng/ml bFGF (PeproTech Co.), 20 ng/ml EGF (PeproTech), with B27^{R} supplement (Thermo Fisher Scientific K.K.).

### (3) Formation of reconstituted skin

A mixture containing 3,000,000 to 6,000,000 human fibroblasts, 3,000,000 to 6,000,000 human epidermal cells and 100 to 1000 human dermal papilla spheroids was allowed to stand in a commercially available cell culture insert (Corning, Inc.), and was cultured for a short period of time in a 37°C incubator until the liquid drained and only cells remained, to prepare a graft.

### (4) Transplanting of graft

Dorsal skin of an SCID individual (Charles River) was cut to a size of ∼1 to 2 cm × ∼1 to 2 cm under anesthesia, and the prepared graft was applied to the incision site and sutured with the surrounding skin. The graft was then secured with a dressing material and bandage. After approximately 10 days the dressing material was removed, and then after about 2 to 3 months the presence of reconstituted skin was confirmed and a photograph was taken with a camera-equipped stereomicroscope. The results showed hair growth at the transplanted part of the graft (Fig. 1).

### <Example 2: Tissue analysis of reconstituted skin>

The reconstituted skin was extracted and fixed overnight with a 4% paraformaldehyde solution, and then a paraffin-embedded tissue block was prepared. After creating a tissue section with a thickness of 4 µm using a microtome, different tissue staining methods were carried out. A hydrophilic treated tissue section was H.&E. stained by a general method, and a photograph was taken with an AxioScan (Zeiss Co.) (Fig. 2).

In order to confirm whether the tissue containing the reconstituted hair follicles was tissue composed of human cells, *in situ* hybridization (ISH) was carried out by a general method using a human genome-specific repeat (Alu repeat) as the probe, and a photograph was taken in the same manner as above (Fig. 3).

Immunostaining with hair shaft-specific antibody AE13 (Abcam Co.) and Alu-ISH double staining were carried out (Fig. 4). The basic methods for immunostaining and ISH were carried out with reference to "Post-genome Research Era Immunostaining/*in situ* Hybridization" (Y odosha).

The results confirmed that the reconstituted skin tissue containing hair follicles was derived from the transplanted human cells. It was also confirmed that hair shafts had been formed.

### <Example 3: Fluorescent-labeled dermal papilla spheroid tracking test>

Dermal papilla cells cultured in the presence of CHIR99021 3uM were labeled with red fluorescent dye PKH-26 (Sigma-Aldrich Japan, KK.) according to the manufacturer's protocol (Fig. 5). Reconstituted skin containing the labeled dermal papilla spheroids was constructed, and as a result, red fluorescence was observed at the dermal papilla sites where hair follicles are formed. Based on this it was concluded that the hair follicles found in reconstituted skin had been induced to form by the dermal papilla spheroids (Fig. 5).

### <Example 4: Confirming the presence of spheroid-formed dermal papilla cells, and their ability to reconstitute hair follicle-containing human skin tissue with and without CHIR99021 stimulation>

Reconstitution of hair follicle-containing human skin tissue in SCID mice was attempted by the method described in Example 1, except that conditions were with or without spheroid-formed dermal papilla cells, and with or without CHIR99021 stimulation. The results are shown below.

**[Table 1]**

| Conditions | Individual with hair follicle-containing human skin |
|---|---|
| Epidermal cells + fibroblasts | 17% (1*/6) |
| Epidermal cells + fibroblasts + spheroid-formed dermal papilla cells | 42% (3/7) |
| Epidermal cells + fibroblasts + spheroid-formed dermal papilla cells + CHIR99021 stimulation | 83% (10/12) |

| | |
|---|---|
| *1: Low number of confirmed hair follicles, epidermal cells partially mouse-derived. | |

## Claims

1. A composition for reconstitution of hair follicle-containing human skin tissue, comprising:
human epidermal cells; and
human dermal cells,
wherein the human dermal cells include a cell population of non-fetal-derived human dermal papilla cells that have undergone spheroid formation.

2. The composition according to claim 1, wherein the non-fetal-derived human dermal papilla cells are non-fetal-derived human dermal papilla cells stimulated with a Wnt signal activating agent.

3. The composition according to claim 2, wherein the Wnt signal activating agent is a Wnt ligand family protein or a Wnt pathway agonist.

4. The composition according to claim 3, wherein the Wnt ligand family protein is selected from the group consisting of Wnt1, Wnt2, Wnt2b, Wnt3, Wnt3a, Wnt4, Wnt5a, Wnt5b, Wnt6, Wnt7a, Wnt7b, Wnt8a, Wnt8b, Wnt9a, Wnt9b, WntlOa, WntlOb, Wnt11, Wnt16 and their combinations.

5. The composition according to claim 3, wherein the Wnt pathway agonist is CHIR99021 or BIO.

6. The composition according to any one of claims 1 to 5, wherein the human dermal cells include human fibroblasts.

7. A hair follicle-containing human skin tissue animal model, wherein a composition according to any one of claims 1 to 6 has been applied to a non-human mammal.

8. A method for producing a composition for reconstitution of hair follicle-containing human skin tissue, wherein the method comprises:
(1) a step of preparing spheroids containing non-fetal-derived human dermal papilla cells, and
(2) a step of mixing and culturing the spheroids, human epidermal cells and human dermal cells.

9. The method according to claim 8, wherein step (1) is carried out in the presence of a Wnt signal activating agent.

10. The method according to claim 9, wherein the Wnt signal activating agent is a Wnt ligand family protein or a Wnt pathway agonist.

11. The method according to claim 10, wherein the Wnt ligand family protein is selected from the group consisting of Wnt1, Wnt2, Wnt2b, Wnt3, Wnt3a, Wnt4, Wnt5a, Wnt5b, Wnt6, Wnt7a, Wnt7b, Wnt8a, Wnt8b, Wnt9a, Wnt9b, WntlOa, WntlOb, Wnt11, Wnt16 and their combinations.

12. The method according to claim 10, wherein the Wnt pathway agonist is CHIR99021 or BIO.

13. The method according to any one of claims 8 to 12, wherein step (1) is carried out by the hanging drop method.

14. The method according to any one of claims 8 to 13, wherein the non-fetal-derived human dermal papilla cells are adult-derived human dermal papilla cells.

15. The method according to any one of claims 8 to 14, wherein the human dermal cells include human fibroblasts.

16. The method according to any one of claims 8 to 15, wherein the human epidermal cells are human epidermal cells stimulated by an extracellular matrix protein or its derivative.

17. The method according to claim 16, wherein the extracellular matrix protein includes laminin or its fragment.

18. The method according to any one of claims 8 to 17, wherein step (2) is a step of culturing on a culture substrate comprising a porous film.

19. A method for producing a hair follicle-containing human skin tissue animal model, comprising:
a step of applying a composition obtained by the method according to any one of claims 8 to 18 to a non-human mammalian animal.

20. A composition for reconstitution of hair follicle-containing human skin tissue, obtained by the method according to any one of claims 8 to 18.

21. A hair follicle-containing human skin tissue animal model obtained by the method according to claim 19.
